# EUROPEAN PATENT SPECIFICATION

(11) **EP 3 916 083 B1**
(45) Date of publication and mention of the grant of the patent: **16.07.2025**
(21) Application number: 21175414.8
(22) Date of filing: 21.05.2021
(51) Int. Cl.: C12N 5/071, C12N 5/0793, C12N 5/079, G01N 33/50

(54) **CELL-CONTAINING VESSEL AND METHOD FOR PRODUCING NEURAL CELL-CONTAINING SPHEROID**
ZELLHALTIGES GEFÄSS UND VERFAHREN ZUR HERSTELLUNG EINES NEURALEN ZELLHALTIGEN SPHÄROIDS
RÉCIPIENT CONTENANT DES CELLULES ET PROCÉDÉ DE PRODUCTION D'UN SPHÉROÏDE CONTENANT DES CELLULES NEURALES

(30) Priority: 27.05.2020 JP 2020092186; 01.04.2021 JP 2021062866
(43) Date of publication of application: 01.12.2021
(73) Proprietor: Ricoh Company, Ltd., Tokyo 143-8555 (JP)
(72) Inventor: IJICHI, Rie, Tokyo, 143-8555 (JP); ARATANI, Tomoyuki, Tokyo, 143-8555 (JP); NAKAYAMA, Tomoaki, Tokyo, 143-8555 (JP); KOSHIZUKA, Shinnosuke, Tokyo, 143-8555 (JP)
(74) Representative: Marks & Clerk LLP

(56) References cited:
- US-A1- 2019 017 097
- SIRENKO OKSANA ET AL: "Functional and Mechanistic Neurotoxicity Profiling Using Human iPSC-Derived Neural 3D Cultures", TOXICOLOGICAL SCIENCES, vol. 167, no. 1, 31 August 2018 (2018-08-31), pages 58 - 76, XP055841651, ISSN: 1096-6080, Retrieved from the Internet <URL:https://www.ncbi.nlm.nih.gov/pmc/articles/PMC6317428/pdf/kfy218.pdf> DOI: 10.1093/toxsci/kfy218
- MCDUFFIE J E ED - VAN RAVENZWAAY BENNARD ET AL: "Early identification of potentially translatable compound-induced neurotoxicity and associated biomarkers utilizing 2D & 3D primary and iPSC-derived microBrain models", TOXICOLOGY LETTERS, ELSEVIER BIOMEDICAL PRESS , AMSTERDAM, NL, vol. 295, 12 September 2018 (2018-09-12), XP085476969, ISSN: 0378-4274, DOI: 10.1016/J.TOXLET.2018.06.1107

## Description

### TECHNICAL FIELD

The present invention relates to a cell-containing vessel and a method for producing a neural cell-containing spheroid. Priorities are claimed on Japanese Patent Applications No. 2020-092186, filed on May 27, 2020, and No. 2021-062866, filed on April 1, 2021.

### BACKGROUND ART

In the research and development of new drugs, in-vitro test systems that use cells are often constructed. For example, in drug screening for cranial nerve disease and toxicity screening for the cranial nervous system, primary culture cells, cells differentiated from undifferentiated ES cells/iPS cells, immortalized cells, and cancer cells and the like are used.

Among the various possibilities, neurons derived from iPS cells can utilize cells derived from the patient, meaning they can reflect the genetic background of the patient, and are therefore attracting much attention due to their ability to construct models having superior extrapolation properties. Further, calcium transient assays is widely used as an assay method capable of viewing neural activity. The application of calcium transient assays using spheroid cultured neurons to toxicity evaluations and drug efficacy evaluations is also being investigated.

For example, Patent Document 1 (International Patent Publication No. 2019/014603) discloses an optical detection method which, in order to conduct functional evaluations with high throughput, includes a step of detecting spheroids of human cells having uniform diameter, a drug, and variations in oscillations.

### SUMMARY OF INVENTION

### Problems to be Solved by the Invention

However, conventional neural cell-containing spheroids have been produced using a method in which astrocytes and neurons are differentially induced simultaneously from pluripotent stem cells such as iPS cells. Specifically, conventionally, nerve cell spheroids containing neurons and astrocytes have been produced by inoculating a spheroid production plate with neural precursor cells produced from pluripotent stem cells and culturing the cells to form spheroids and induce differentiation into neurons and astrocytes using the medium components. However, differentiation induction systems that use neural precursor cells exhibit poor differentiation synchrony, and obtaining uniform cell groups tends to be difficult.

Consequently, the proportion of neural cells contained in the spheroids differs depending on the differentiation induction state at the time, such as the state of the neural precursor cells, and cannot always be controlled at a constant level, meaning a problem arises in that the variation between samples is large and the reproducibility is poor.

Accordingly, the present invention has an object of providing a technique for suppressing functional variations between a plurality of neural cell-containing spheroids across a plurality of lots or within a single lot.

### Means for Solving the Problems

A neural cell-containing vessel contains a plurality of neural cell-containing spheroids for which the variation calculated using the calculation method described below is less than 20%, wherein the neural cell-containing spheroids contain a plurality of types of cells including neural cells.

### (Calculation Method)

Calcium transient assays are conducted for each of a plurality of neural cell-containing spheroids, the number of spontaneous oscillations in a 10-minute period is measured, the average and standard deviation for the number of spontaneous oscillations are calculated, and the variation is calculated using formula (1) below. Variation (%) = standard deviation for number of spontaneous oscillations / average number of spontaneous oscillations × 100 ... (1)

A method for producing a neural cell-containing spheroid according to the present invention includes the steps of: mixing neurons for which the cell lineage has been determined and astrocytes for which the cell lineage has been determined in a predetermined ratio of cell number of neurons : cell number of astrocytes, wherein the predetermined ratio is within a range from 0.8 : 1 to 1.2 : 1; and culturing a mixture obtained in the mixing step, thereby forming a spheroid.

### Effects of the Invention

The present invention is able to provide a technique for suppressing functional variations between a plurality of neural cell-containing spheroids across a plurality of lots or within a single lot.

### BRIEF DESCRIPTION OF THE DRAWINGS

FIG. 1 is a schematic diagram describing a conventional method for producing neural cell-containing spheroids.
FIG. 2 is a schematic diagram describing a method for producing neural cell-containing spheroids according to an embodiment of the present invention.
FIG. 3 is a schematic diagram describing neural cell-containing spheroids according to an embodiment of the present invention.
FIG. 4 is a schematic diagram describing neural cell-containing spheroids from a plurality of lots obtained using a conventional production method.
FIG. 5 is a schematic diagram describing neural cell-containing spheroids from a plurality of lots according to an embodiment of the present invention.
FIG. 6 is a graph illustrating one example of spontaneous oscillations observed in a calcium transient assay.
FIGS. 7(a) to (f) are graphs illustrating the results of calcium transient assays of three representative spheroids from test numbers 1-1 to 1-6 respectively in Test Example 1.
FIG. 8 is a graph summarizing the numbers of spontaneous oscillations in a 10-minute period based on the results of calcium transient assays for 24 spheroids in Test Example 1.
FIG. 9 is a graph illustrating the diameters of the spheroids in test numbers 1-1 to 1-6 in Test Example 1.
FIG. 10(a) and (b) are graphs illustrating the results of calcium transient assays of three representative spheroids from test numbers 2-1 and 2-2 respectively in Test Example 2.
FIG. 11 is a graph summarizing the numbers of spontaneous oscillations in a 10-minute period based on the results of calcium transient assays for 18 spheroids in Test Example 2.
FIG. 12 is a graph illustrating the diameters of the spheroids in test numbers 2-1 to 2-3 in Test Example 1.
FIG. 13(a) and (b) are graphs illustrating the results of calcium transient assays of three representative spheroids from test numbers 3-1 and 3-2 respectively in Test Example 3.
FIG. 14 is a series of fluorescence microscope photographs illustrating the results of immunostaining in Test Example 4.

### EMBODIMENTS FOR CARRYING OUT THE INVENTION

### [Method for Producing Neural Cell-Containing Spheroid]

In one embodiment, the present invention provides a method for producing a neural cell-containing spheroid, the method including the steps of: mixing neurons for which the cell lineage has been determined and astrocytes for which the cell lineage has been determined in a predetermined ratio of cell number of neurons : cell number of astrocytes, wherein the predetermined ratio is within a range from 0.8 : 1 to 1.2 : 1; and culturing a mixture obtained in the mixing step, thereby forming a spheroid.

As described in the examples below, among neural cell-containing spheroids produced using the production method of this embodiment, functional variations between a plurality of neural cell-containing spheroids are suppressed across a plurality of lots or within a single lot.

In this description, a lot describes products produced at the same time under the same conditions. The expression that "functional variations between a plurality of neural cell-containing spheroids are suppressed across a plurality of lots" means that when neural cell-containing spheroids representing each lot are extracted randomly from the plurality of lots of neural cell-containing spheroids, the functional variations between these neural cell-containing spheroids are minor.

Further, the expression that "functional variations between a plurality of neural cell-containing spheroids are suppressed within a single lot" means that when a plurality of neural cell-containing spheroids are extracted randomly from a single lot of neural cell-containing spheroids, the functional variations between these neural cell-containing spheroids are minor.

A function of a neural cell-containing spheroid refers to an activity of the neural cell-containing spheroid indicated by a value measured using some form of assay. For example, the number of spontaneous oscillations in a predetermined period, measured by supplying the neural cell-containing spheroid to a calcium transient assay, may be used. Alternatively, the responsiveness to a drug may be used. Examples of the responsiveness to a drug include, but are not limited to, a change in the spontaneous oscillations in the presence of the drug, or a change in the expression level of a predetermined gene.

The expression that "functional variations are minor" means, for example, that the variation calculated using the calculation method described below is, for example, less than 20%, and may be less than 15% or less than 10%.

### (Calculation Method)

A plurality of neural cell-containing spheroids from across a plurality of lots or from within a single lot are each subjected to a calcium transient assay, the number of spontaneous oscillations in a 10-minute period is measured, the average and standard deviation for the number of spontaneous oscillations are calculated, and the variation is calculated using formula (1) below. Variation (%) = standard deviation for number of spontaneous oscillations / average number of spontaneous oscillations × 100

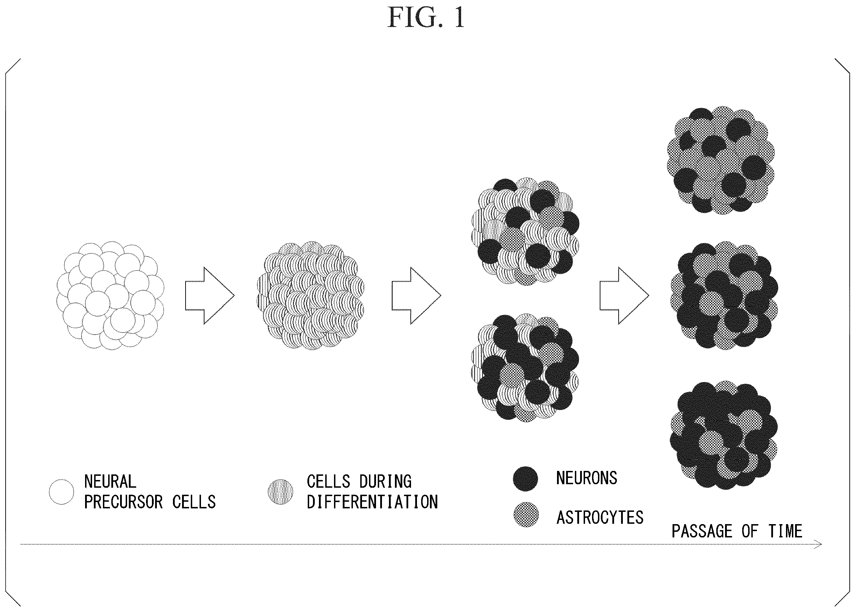

FIG. 1 is a schematic diagram describing a conventional method for producing neural cell-containing spheroids. As illustrated in FIG. 1, in this conventional production method, spheroids are formed using neural precursor cells for which the cell lineage has not been determined. Consequently, differentiation induction occurs together with spheroid formation. Accordingly, the cells constituting the spheroids change over time, and as a result of differentiation induction, spheroids containing neurons and astrocytes are obtained. In this production method, uniform cell groups cannot be obtained, and the obtained spheroids include the neurons and astrocytes in various proportions.

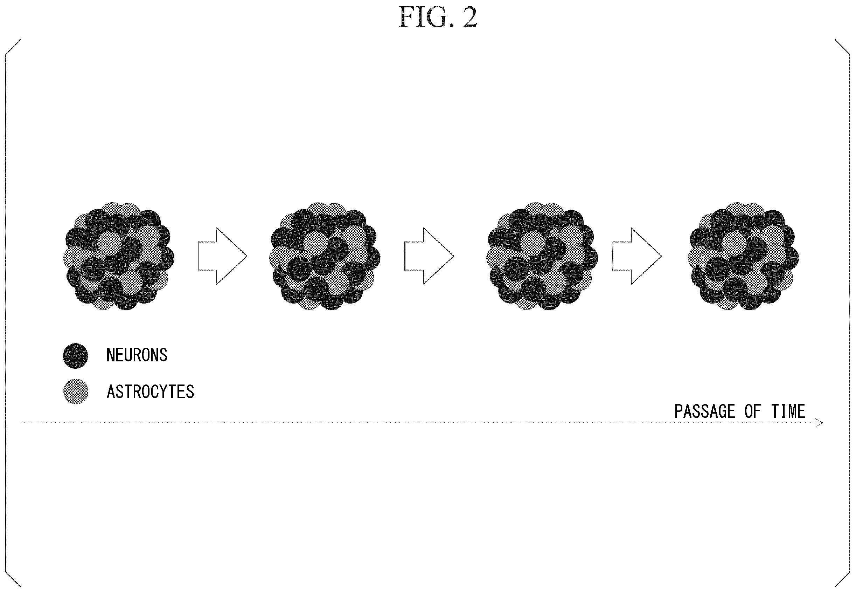

FIG. 2 is a schematic diagram describing the production method of the present embodiment. As illustrated in FIG. 2, in the production method of the present embodiment, neural cells for which the cell lineage has been determined are mixed in a predetermined ratio to form the spheroids. In the example of FIG. 2, the neural cell-containing spheroid contains differentiated neurons and differentiated astrocytes. Accordingly, the proportions of the types of neural cells that constitute the spheroid do not change over time.

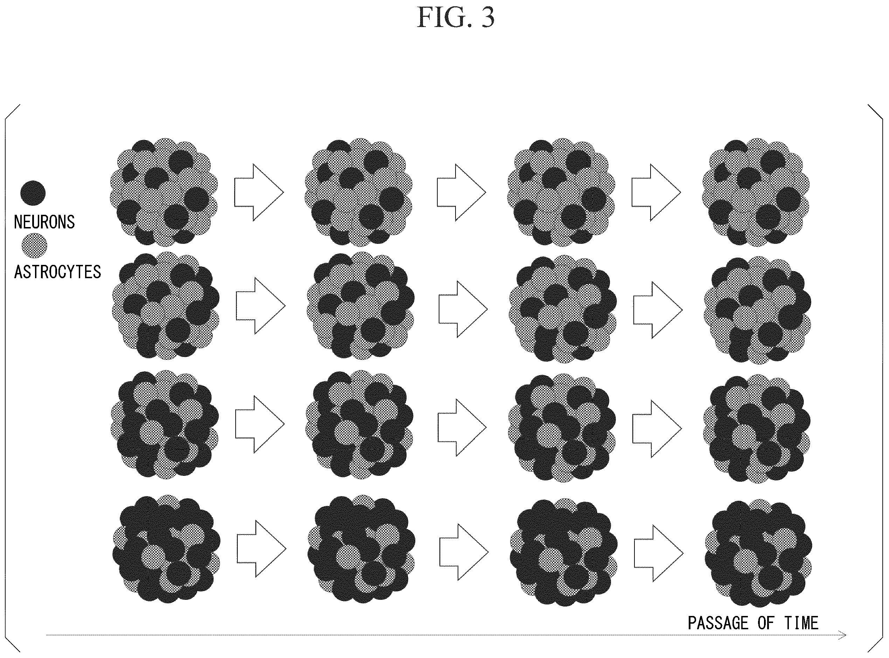

Further, as illustrated in FIG. 3, in the production method of the present embodiment, by changing the proportions of the neural cells that are mixed, neural cell-containing spheroids containing a plurality of types of neural cells in a predetermined ratio can be produced.

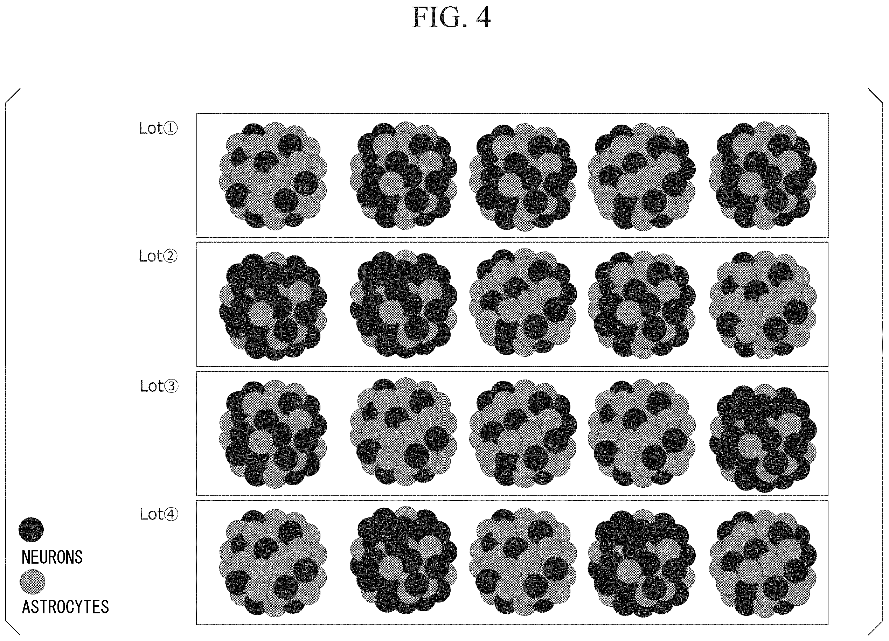

FIG. 4 is a schematic diagram describing neural cell-containing spheroids from a plurality of lots obtained using a conventional production method. As illustrated in FIG. 4, the neural cell-containing spheroids obtained using a conventional production method exhibit a large variation in the proportions of the types of constituent neural cells, both for a plurality of neural cell-containing spheroids across a plurality of lots, and for a plurality of neural cell-containing spheroids from within a single lot. As a result, functional variations between the various neural cell-containing spheroids are large.

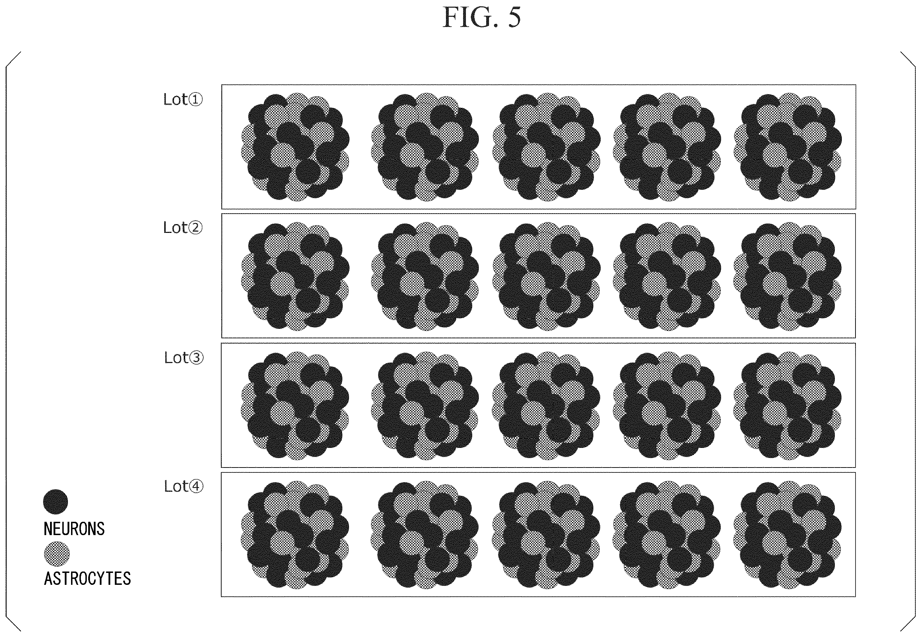

FIG. 5 is a schematic diagram describing neural cell-containing spheroids from a plurality of lots obtained using the production method of the present embodiment. As illustrated in FIG. 5, because the neural cell-containing spheroids obtained using the production method of the present embodiment use neural cells for which the cell lineage has been determined, the proportions of the types of neural cells contained in the neural cell-containing spheroids can be maintained at a constant level. Accordingly, variations in the proportions of the types of constituent neural cells are minor, both for a plurality of neural cell-containing spheroids across a plurality of lots, and for a plurality of neural cell-containing spheroids from within a single lot. As a result, functional variations between the various neural cell-containing spheroids are also minor.

In the production method of the present embodiment, the neural cells for which the cell lineage has been determined may be differentiated neural cells, or stem cells destined for differentiation into neural cells. The differentiated neural cells may be primary cells extracted from a living body, or neural cells differentially induced from stem cells. Further, the stem cells may be pluripotent stem cells or neural precursor cells.

Examples of pluripotent stem cells include embryonic stem cells (ES cells) and induced pluripotent stem cells. Examples of induced pluripotent stem cells include induced pluripotent stem cells (iPS cells). Among these, iPS cells are preferred as the pluripotent stem cells.

The iPS cells may be derived from a healthy subject, or derived from a patient having any of various neural disorders. Further, the iPS cells may have been subjected to any of various forms of gene editing, and for example, may be cells that have been subjected to gene editing so as to have genes that either cause, or are risk factors for, any of various neural disorders.

**In** those cases where the iPS cells are derived from a patient having any of various neural disorders, the cells can be used for constructing a model for the neural disorder. There are no particular limitations on the neural disorder, and examples include neurodegenerative diseases, autism, epilepsy, attention-deficit hyperactivity disorder (ADHD), schizophrenia, and bipolar disorder. Examples of neurodegenerative diseases include Alzheimer's disease, Parkinson's disease, and amyotrophic lateral sclerosis.

There are no particular limitations on the animal from which the neural cells are derived, and examples include humans, monkeys, dogs, cows, horses, sheep, pigs, rabbits, mice, rats, marmots and hamsters. Among these, humans are preferred.

Examples of the neural cells include neurons and astrocytes.

Neurons can, for example, be broadly classified into peripheral neurons and central neurons. Examples of peripheral neurons include sensory neurons, motor neurons and autonomic neurons. Examples of central neurons include interneurons and projection neurons. Examples of these projection neurons include cortical neurons, hippocampal neurons and amygdala neurons. Further, the central neurons can be broadly classified into excitatory neurons and inhibitory neurons. Examples include glutamatergic neurons that carry mainly excitatory transmissions in the central nervous system, and GABA (γ-aminobutyric acid)-ergic neurons that carry mainly inhibitory transmissions.

In addition, examples of neurons that release neuromodulators include cholinergic neurons, dopaminergic neurons, noradrenergic neurons, serotonergic neurons, and histaminergic neurons.

Further, the astrocytes may be primary culture cells, or cells differentially induced from stem cells.

The neural cells for which the cell lineage has been determined may be pluripotent stem cells that have undergone a differentiation induction treatment to form neural cells. An example of the differentiation induction treatment involves introducing a specific transcription factor into pluripotent stem cells. Specific examples of such cells include cells from the Quick-Neuron (TM) series manufactured by Elixirgen Scientific, Inc. These cells are pluripotent stem cells that have undergone a differentiation induction treatment into neural cells, and have been differentiated into functionally mature neural cells over an approximately 10-day period.

The neural cells for which the cell lineage has been determined may also be cells other than neural cells that have undergone a differentiation induction treatment to form neural cells. The differentiation induction treatment is as described above. Examples of the cells other than neural cells include skeletal muscle cells, vascular endothelial cells and blood cells. Examples of the animals that function as the source for the cells other than neural cells include, but are not limited to, humans, monkeys, dogs, cows, horses, sheep, pigs, rabbits, mice, rats, marmots and hamsters. Among these, humans are preferred. Furthermore, the cells other than neural cells may be primary culture cells, or cells differentially induced from stem cells.

Following mixing of the plurality of types of neural cells in the predetermined ratio, in which the cell lineage of the neural cells has been determined, the cells are inoculated and cultured in a suitable cell culture vessel such as a spheroid production plate to produce spheroids. The period for which the cell culture vessel is incubated may be set appropriately in accordance with the intended purpose, but the period is preferably sufficient to ensure that the neural cells mature to a level that enables their functionality to be evaluated.

For example, in the case where pluripotent stem cells that have undergone a differentiation induction treatment to form neural cells are used as the neural cells for which the cell lineage has been determined, the time period from the point where the pluripotent stem cells that have undergone a differentiation induction treatment to form a plurality of types of neural cells are mixed and inoculated may be, for example, at least 20 days, at least 30 days, at least 40 days, at least 50 days, at least 60 days, or 70 days or longer.

A medium prepared by adding essential components to a basal medium may be used as the medium. Examples of the basal medium include BrianPhys (manufactured by STEMCELL Technologies Inc.), Neurobasal (manufactured by Thermo Fisher Scientific Inc.), Dulbecco's Modified Eagle's Medium (DMEM), Ham's Nutrient Mixture F12, D-MEM/F12 Medium, McCoy's 5A medium, Eagle's Minimum Essential Medium (EMEM), alpha Modified Eagle's Minimum Essential Medium (αMEM), Minimum Essential Medium (MEM), Rosewell Park Memorial Institute-1640 (RPMI-1640), Iscove's Modified Dulbecco's Medium (IMDM), MCDB131 Medium, Williams' Medium E, IPL41 Medium, Fischer's Medium, M199 Medium, High Performance Medium 199, StemPro 34 (manufactured by Thermo Fisher Scientific Inc.), X-VIVO 10 (manufactured by Chembrex Ltd.), X-VIVO 15 (manufactured by Chembrex Ltd.), HPGM (manufactured by Chembrex Ltd.), StemSpan H3000 (manufactured by STEMCELL Technologies Inc.), StemSpan SFEM (manufactured by STEMCELL Technologies Inc.), StemlineII (manufactured by Sigma Aldrich Co., Ltd.), QBSF-60 (manufactured by Quality Biological Inc.), StemPro hESC SFM (manufactured by Thermo Fisher Scientific Inc.), Essential 18 (a registered trademark) Medium (manufactured by Thermo Fisher Scientific Inc.),mTeSR1 or mTeSR2 Medium (manufactured by STEMCELL Technologies Inc.), Repro FF or Repro FF2 (manufactured by REPROCELL Inc.), PSGro hESC/iPSC Medium (manufactured by System Biosciences, LLC), NutriStem (a registered trademark) Medium (manufactured by Biological Industries Co., Ltd.), CSTI-7 Medium (manufactured by Cell Science & Technology Institute, Inc.), MesenPRO RS Medium (manufactured by Thermo Fisher Scientific Inc.), MF-Medium (a registered trademark) (a mesenchymal stem cell proliferation medium, manufactured by Toyobo Co., Ltd.), Sf-900II (manufactured by Thermo Fisher Scientific Inc.), and Opti-Pro (manufactured by Thermo Fisher Scientific Inc.). One of these media may be used alone, or a mixture of two or more media may be used.

Further, examples of additives that may be added to the basal medium include the types of additives typically used in the culturing of neurons, with specific examples including SM1 Supplement (manufactured by STEMCELL Technologies Inc.), N2 Supplement A (manufactured by STEMCELL Technologies Inc.), Rat Astrocyte Culture Supernatant (manufactured by FUJIFILM Wako Pure Chemical Corporation), Human Astrocyte Culture Supernatant (manufactured by ScienCell Research Laboratories, Inc.), Component N (manufactured by Elixirgen Scientific, Inc.), Component G2 (manufactured by Elixirgen Scientific, Inc.), Component P (manufactured by Elixirgen Scientific, Inc.), N2 Supplement (manufactured by Thermo Fisher Scientific Inc.), iCell Neural Supplement B (manufactured by CDI, Inc.), and iCell Nervous System Supplement B-27 plus (manufactured by Thermo Fisher Scientific Inc.).

Next is a description of a calcium transient assay. In a calcium transient assay, the neural cell-containing spheroids are first prepared. For example, the desired number of neurons and astrocytes are mixed, the mixture is inoculated onto a spheroid production plate, and culturing is performed for 3 to 8 weeks in a medium suitable for the neuron culturing, with the medium replaced as appropriate.

Next, the spheroids are transferred to a plate suitable for optical detection, and a calcium-sensitive fluorescent dye is added to the medium. The calcium-sensitive fluorescent dye is a substance which is incorporated into the cells and emits fluorescence upon binding to a calcium ion. Examples of substances that may be used appropriately as the calcium-sensitive fluorescent dye include Cal-520, Fluo4, Calcium-6 and EarlyTox Cardiotoxicity Kit (manufactured by Molecular Devices, LLC), and Cal-520 AM (manufactured by AAT Bioquest, Inc. (formerly ABD Bioquest, Inc.)).

Subsequently, an excitation wavelength and a fluorescence wavelength suitable for the calcium-sensitive fluorescent dye being used are set in a device capable of detecting fluorescence, and measurement is conducted continuously for a predetermined time. Examples of devices that may be used include a fluorescence plate reader, FDSS (Functional Drug Screening System, manufactured by Hamamatsu Photonics K.K.), and FLIPR (Fluorometric Imaging Plate Reader, manufactured by Molecular Devices, LLC). Measurements may, for example, be conducted every 0.5 seconds for a period of 10 minutes.

Neurons performing neural activity undergo repeated spontaneous uptake and release of calcium ions. Accordingly, when a calcium transient assay is conducted using the neural cell-containing spheroids, the fluorescence intensity varies periodically and is measured as spontaneous oscillations.

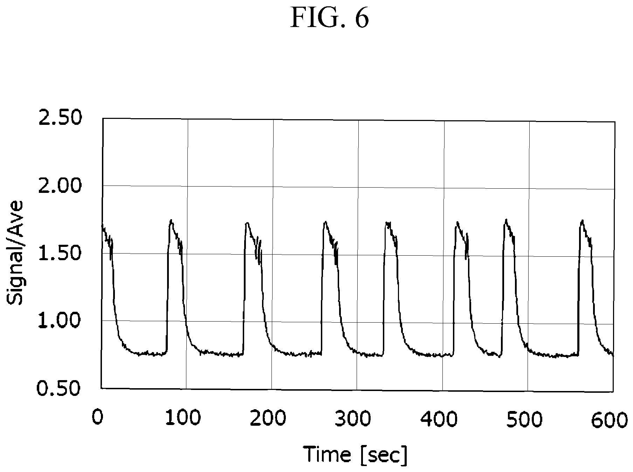

FIG. 6 is a graph illustrating one example of spontaneous oscillations observed in a calcium transient assay. The horizontal axis in the graph indicates the time (seconds). Further, the vertical axis indicates the measured fluorescence intensity as a ratio of the fluorescence intensity divided by the fluorescence intensity observed immediately following the start of measurements.

### [Neural Cell-Containing Spheroids]

In one embodiment, the present invention provides neural cell-containing spheroids which contain a plurality of types of differentiated neural cells in a predetermined mixing ratio, and for which the variation calculated in accordance with the calculation method described below is less than 20%.

### (Calculation Method)

Calcium transient assays are conducted for each of a plurality of neural cell-containing spheroids from across a plurality of lots or from within a single lot, the number of spontaneous oscillations in a 10-minute period is measured, the average and standard deviation for the number of spontaneous oscillations are calculated, and the variation is calculated using formula (1) below. Variation (%) = standard deviation for number of spontaneous oscillations / average number of spontaneous oscillations × 100

The neural cell-containing spheroids of the present embodiment can be produced using the production method described above.

The variation calculated using the above formula (1) may, for example, be less than 15%, or may be less than 10%.

For the neural cell-containing spheroids of the present embodiment, the variation calculated in accordance with the calculation method described below is preferably less than 15%, more preferably less than 10%, and particularly preferably less than 5%.

### (Calculation Method)

For each of a plurality of neural cell-containing spheroids from across a plurality of lots or from within a single lot, the cell number of neurons and the cell number of astrocytes are measured, the average and standard deviation for the ratio of the cell number of neurons relative to the cell number of astrocytes (cell number of neurons / cell number of astrocytes) are calculated, and the variation is calculated using formula (2) below. Variation (%) = standard deviation for (cell number of neurons / cell number of astrocytes) / average for (cell number of neurons / cell number of astrocytes) × 100

In the above formula (2), the cell number of neurons and the cell number of astrocytes can be measured, for example, by subjecting the spheroids to oral immunostaining to stain the neurons and the astrocytes, and then inspecting the spheroids under an optical microscope. Examples of neuron markers that may be used include MAP2, Tubulin beta 3, NeuN, 160 kDa Neurofilament, 200 kDa Neurofilament, NSE, PSD93, and PSD95. Examples of astrocyte markers include GFAP, S100β, Cx43, EAAT1, EAAT2, Glutamine synthetase, and ALDH1L1.

Alternatively, the cell number of the neurons and astrocytes in the spheroids may be calculated, without actually measuring cell number, by treating the total fluorescence intensity of each type of stained cell as a value that corresponds with the cell number, and calculating the variation using these fluorescence intensity values. Alternatively, the variation may also be calculated by treating the surface area or volume occupied by each type of stained cell in a microscope image as a value that corresponds with the cell number.

In these cases, the variation may be calculated using formula (3) shown below instead of the above formula (2). Variation (%) = standard deviation for (value corresponding with cell number of neurons / value corresponding with cell number of astrocytes) / average for (value corresponding with cell number of neurons / value corresponding with cell number of astrocytes) × 100

In the neural cell-containing spheroids of the present embodiment, the ratio of the cell number of neurons relative to the cell number of astrocytes (or value corresponding with cell number of neurons / value corresponding with cell number of astrocytes) may be approximately 1.

As described below in the examples, when the value of cell number of neurons / cell number of astrocytes is 1, namely when the ratio of cell number of neurons : cell number of astrocytes is approximately 1:1, functional variations for the neural cell-containing spheroids may sometimes be less than in the case where the ratio of cell number of neurons : cell number of astrocytes is approximately 2:1 or approximately 4:1.

The value of cell number of neurons / cell number of astrocytes need not be exactly 1, provided it is within a range from 0.8 to 1.2, for example from 0.9 to 1.1. Further, it is though that depending on the neurons and astrocytes used, the proportion of each of the cells at which functional variations for the neural cell-containing spheroids are reduced may vary.

### [Cell-Containing Vessel]

Herein disclosed is a cell-containing vessel that contains the neural cell-containing spheroids produced in the production method described above. There are no particular limitations on the form of the vessel, and examples include a tube or a multi-well plate. Examples of the multi-well plate include multi-well plates having 24 wells, 48 wells, 96 wells, 384 wells or 1,536 wells.

There are no particular limitations on the shape, volume, material, or color or the like of the wells in the multi-well plate, and these may be selected as appropriate.

There are no particular limitations on the well shape, provided the well is capable of holding the spheroids, and the shape may be selected appropriately in accordance with the intended purpose. For example, wells having a flat bottom, round bottom, U-shaped bottom or V-shaped bottom may be used.

There are no particular limitations on the volume of each well, which may be selected appropriately in accordance with the intended purpose, but for example, if consideration is given to the amount of reagent used in typical evaluation methods, then the volume may be within a range from 5 to 1,000 µL, from 30 to 300 µL, or from 50 to 200 µL.

Examples of the color of the multi-well plate include transparent, semitransparent, colored, and totally opaque plates. Further, when conducting evaluations using an optical system, from the viewpoint of suppressing interference from neighboring wells, a vessel in which the bottom portion is transparent and the side wall portions are colored is preferred.

The material of the multi-well plate may be selected appropriately in accordance with the intended purpose, and examples include organic materials, including polyethylene terephthalate (PET), polystyrene (PS), polycarbonate (PC), TAC (triacetyl cellulose), polyimide (PI), nylon (Ny), low-density polyethylene (LDPE), mediumdensity polyethylene (MDPE), vinyl chloride, vinylidene chloride, polyphenylene sulfide, polyethersulfone, polyethylene naphthalate, polypropylene, acrylic-based materials such as urethane acrylate, cellulose and polydimethylsiloxane (PDMS), and inorganic materials such as glass and ceramics.

In order to prevent the spheroids from adhering to the bottom surface and side surfaces of the multi-well plate, the surface of the multi-well plate is preferably subjected to an ultra-low adhesion treatment. Examples of such treatments include coating with a polymer having phosphorylcholine groups, a covalent bonding treatment with a neutrally charged hydrophilic gel, and coating with a synthetic polymer having a similar structure to the polar group of phosphatidylcholine. Provided cell adhesion can be suppressed, the treatment is not limited to these treatments, and any appropriate treatment may be selected.

The vessel of the present embodiment may be a multi-well plate in which each well contains one neural cell-containing spheroid. This type of vessel is convenient, as it can be used without modification in various assays.

Because the vessel of the present embodiment contains neural cell-containing spheroids having reduced functional variation, when the vessel is used for drug efficacy evaluations or toxicity evaluations, variations are minor, and data having high reproducibility can be obtained.

### [Test Substance Evaluation Method]

A method for evaluating the efficacy or toxicity of a test substance includes a step of evaluating the functionality of neural cell-containing spheroids in the presence of the test substance, wherein the neural cell-containing spheroids contain a plurality of types of differentiated neural cells in a predetermined mixing ratio, and exhibit a variation calculated in accordance with the calculation method described below of less than 20%.

### (Calculation Method)

A plurality of neural cell-containing spheroids from across a plurality of lots or from within a single lot are each subjected to a calcium transient assay, the number of spontaneous oscillations in a 10-minute period is measured, the average and standard deviation for the number of spontaneous oscillations are calculated, and the variation is calculated using formula (1) below. Variation (%) = standard deviation for number of spontaneous oscillations / average number of spontaneous oscillations × 100

The evaluation method may also be used as a drug screening method. There are no particular limitations on the test substance, and examples include substances from the natural compound library, synthetic compound library, existing drug library or metabolite library.

### EXAMPLES

The present invention is described below in further detail using a series of examples, but the present invention is not limited to the following examples.

### [Test Example 1]

### (Production of Spheroids using Human iPSC-Derived Neurons and Human-Derived Primary Culture Astrocytes)

Human iPSC-derived neurons (mixed neurons, manufactured by Elixirgen Scientific, Inc.) and human-derived primary culture astrocytes (manufactured by Thermo Fisher Scientific Inc.) were mixed so as to achieve cell numbers per well that satisfied the cell numbers indicated below in Table 1 for test numbers 1-1 to 1-6. Subsequently, each cell mixture was inoculated onto a spheroid production plate and cultured to produce a spheroid. A medium prepared by adding SM1 Supplement (manufactured by STEMCELL Technologies Inc.), N2 Supplement A (manufactured by STEMCELL Technologies Inc.) and a 10% astrocyte culture supernatant (manufactured by ScienCell Research Laboratories, Inc.) to BrianPhys (manufactured by STEMCELL Technologies Inc.) was used as the medium.

Subsequently, following culturing for at least 4 weeks, each spheroid was subjected to a calcium transient assay. Specifically, each spheroid was transferred to a measurement plate, a calcium-sensitive fluorescent dye (EarlyTox Cardiotoxicity Kit, manufactured by Molecular Devices, LLC) was added to the medium, and the mixture was incubated. A fluorescence plate reader was then used to measure the fluorescence at 0.6-second intervals for a 10-minute period.

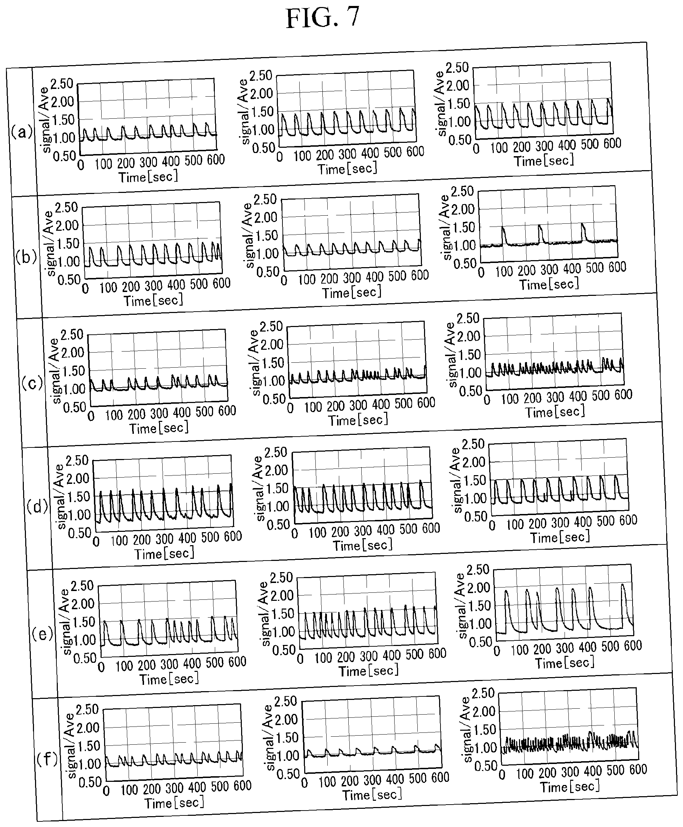

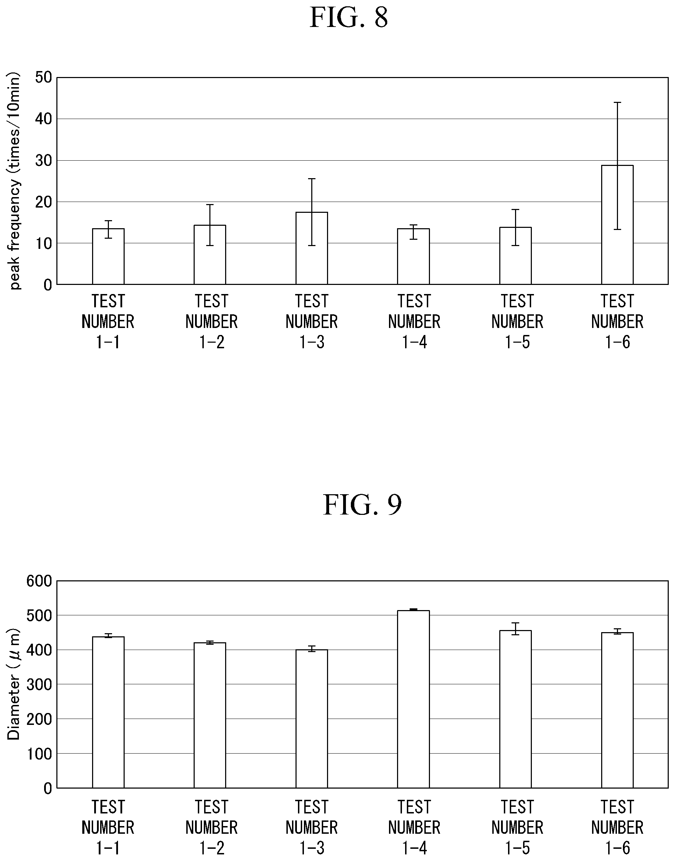

FIGS. 7(a) to (f) are graphs illustrating the measurement results for three representative spheroids from test numbers 1-1 to 1-6 respectively. In FIGS. 7(a) to (f), the horizontal axis of the graph indicates the time (seconds). Further, the vertical axis indicates the measured fluorescence intensity as a ratio of the fluorescence intensity divided by the fluorescence intensity observed immediately following the start of measurements. Further, FIG. 8 is a graph summarizing the numbers of spontaneous oscillations in a 10-minute period based on the results of calcium transient assays for 24 spheroids. In FIG. 8, the graph values indicate the average value ± the standard deviation.

Table 1 below shows the results of calculating the variation using formula (1) shown below, based on the average value and standard deviation for the number of spontaneous oscillations. Variation (%) = standard deviation for number of spontaneous oscillations / average number of spontaneous oscillations × 100

**[Table 1]**

| Test number | Neurons (number) | Astrocytes (number) | Cell number ratio (neurons : astrocytes) | Variation (%) |
|---|---|---|---|---|
| 1-1 | 4.0 × 10³ | 4.0 × 10³ | 1:1 | 15.2 |
| 1-2 | 4.0 × 10³ | 2.0 × 10³ | 2:1 | 34.5 |
| 1-3 | 4.0 × 10³ | 1.0 × 10³ | 4:1 | 45.1 |
| 1-4 | 8.0 × 10³ | 8.0 × 10³ | 1:1 | 13.8 |
| 1-5 | 8.0 × 10³ | 4.0 × 10³ | 2:1 | 32.3 |
| 1-6 | 8.0 × 10³ | 2.0 × 10³ | 4:1 | 52.8 |

Based on the results, it was clear that a variation of less than 20% (reproducibility of 80% or higher) was able to be achieved when the cell number ratio (neurons : astrocytes) was 1: 1.

Subsequently, following culturing for 8 weeks, an image of each spheroid captured using a phase difference microscope was analyzed using software (name: ImageJ, https://imagej.nih.gov/ij/), and the diameter of the spheroid was calculated. FIG. 9 is a graph illustrating the diameters of the spheroids produced under each of the various conditions. In FIG. 9, the graph values indicate the average value ± the standard deviation.

### [Test Example 2]

### (Production of Spheroids using Human iPSC-Derived Neurons and Human iPSC-Derived Astrocytes)

Human iPSC-derived neurons (mixed neurons, manufactured by Elixirgen Scientific, Inc.) and human iPSC-derived astrocytes (Human iPS-derived Astrocytes (mature), manufactured by Xcell Science Inc.) were mixed so as to achieve cell numbers per well that satisfied the cell numbers indicated below in Table 2 for test numbers 2-1 and 2-2. Subsequently, each cell mixture was inoculated onto a spheroid production plate and cultured to produce a spheroid. A medium prepared by adding SM1 Supplement (manufactured by STEMCELL Technologies Inc.) and a 10% astrocyte culture supernatant (manufactured by ScienCell Research Laboratories, Inc.) to BrianPhys (manufactured by STEMCELL Technologies Inc.) was used as the medium.

Subsequently, following culturing for at least 4 weeks, each spheroid was subjected to a calcium transient assay. Specifically, each spheroid was transferred to a measurement plate, a calcium-sensitive fluorescent dye (EarlyTox Cardiotoxicity Kit, manufactured by Molecular Devices, LLC) was added to the medium, and the mixture was incubated. A fluorescence plate reader was then used to measure the fluorescence at 0.6-second intervals for a 10-minute period.

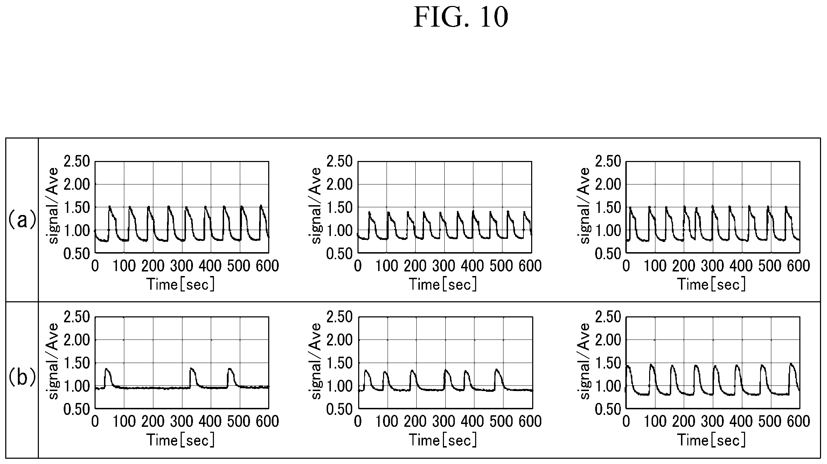

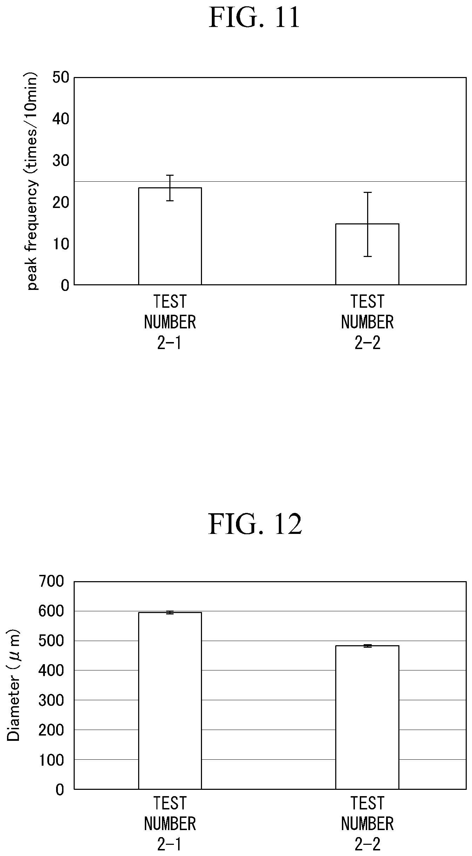

FIGS. 10(a) and (b) are graphs illustrating the measurement results for three representative spheroids from test numbers 2-1 and 2-2 respectively. In FIGS. 10(a) and (b), the horizontal axis of the graph indicates the time (seconds). Further, the vertical axis indicates the measured fluorescence intensity as a ratio of the fluorescence intensity divided by the fluorescence intensity observed immediately following the start of measurements. Further, FIG. 11 is a graph summarizing the numbers of spontaneous oscillations in a 10-minute period based on the results of calcium transient assays for 18 spheroids. In FIG. 11, the graph values indicate the average value ± the standard deviation.

Table 2 below shows the results of calculating the variation using formula (1) shown below, based on the average value and standard deviation for the number of spontaneous oscillations. Variation (%) = standard deviation for number of spontaneous oscillations / average number of spontaneous oscillations × 100

**[Table 2]**

| Test number | Neurons (number) | Astrocytes (number) | Cell number ratio (neurons : astrocytes) | Variation (%) |
|---|---|---|---|---|
| 2-1 | 8.0 × 10³ | 8.0 × 10³ | 1:1 | 13.4 |
| 2-2 | 8.0 × 10³ | 2.0 × 10³ | 4:1 | 53.5 |

Based on the results, it was clear that a variation of less than 20% (reproducibility of 80% or higher) was able to be achieved when the cell number ratio (neurons : astrocytes) was 1: 1.

Subsequently, following culturing for 7 weeks, an image of each spheroid captured using a phase difference microscope was analyzed using software (name: ImageJ, https://imagej.nih.gov/ij/), and the diameter of the spheroid was calculated. FIG. 12 is a graph illustrating the diameters of the spheroids produced under each of the various conditions. In FIG. 12, the graph values indicate the average value ± the standard deviation.

### [Test Example 3]

### (Investigation of Drug Responsiveness using Spheroids)

Spheroids were produced using human iPSC-derived neurons and human iPSC-derived astrocytes, and the drug responsiveness was investigated. First, human iPSC-derived neurons (mixed neurons, manufactured by Elixirgen Scientific, Inc.) and human iPSC-derived astrocytes (Human iPS-derived Astrocytes (mature), manufactured by Xcell Science Inc.) were mixed so as to achieve cell numbers per well that satisfied the cell numbers indicated below in Table 3 for test numbers 3-1 and 3-2. Subsequently, each cell mixture was inoculated onto a spheroid production plate and cultured to produce a spheroid. A medium prepared by adding SM1 Supplement (manufactured by STEMCELL Technologies Inc.) and a 10% astrocyte culture supernatant (manufactured by ScienCell Research Laboratories, Inc.) to BrianPhys (manufactured by STEMCELL Technologies Inc.) was used as the medium.

**[Table 3]**

| Test number | Neurons (number) | Astrocytes (number) | Cell number ratio (neurons : astrocytes) |
|---|---|---|---|
| 3-1 | 8.0 × 10³ | 8.0 × 10³ | 1:1 |
| 3-2 | 8.0 × 10³ | 2.0 × 10³ | 4:1 |

Subsequently, following culturing for 7 weeks, each of the spheroids was subjected to a calcium transient assay in the presence of a drug. The calcium channel blocker 4-aminopyridine (4-AP) was used as the drug. It is known that excessive excitation is induced in neurons by 4-AP. In the calcium transient assays, excessive excitation of the neurons was detected as an increase in oscillation frequency or an elevated baseline.

Specifically, first, each spheroid was transferred to a measurement plate, a calcium-sensitive fluorescent dye (EarlyTox Cardiotoxicity Kit, manufactured by Molecular Devices, LLC) was added to the medium, and the mixture was incubated. Subsequently, 4-AP was added to the medium in an amount sufficient to achieve a final concentration of 30 µM, and fluorescence measurements were conducted at 0.6-second intervals for the 10-minute period immediately following the addition.

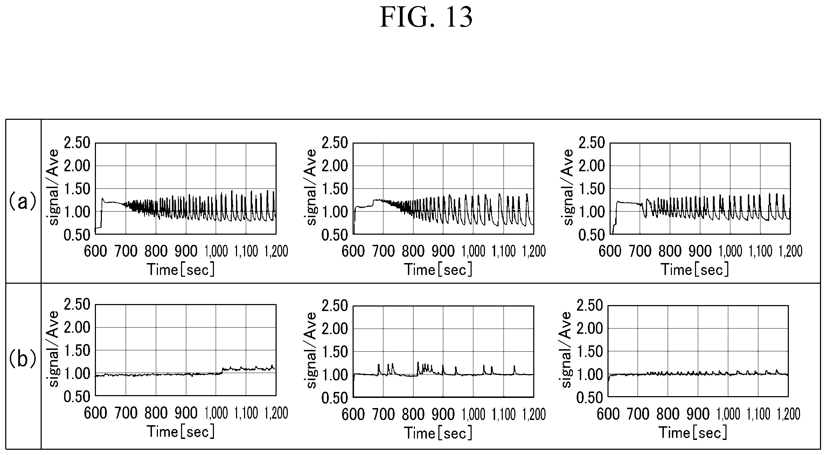

FIG. 13(a) and (b) are graphs illustrating the measurement results for three representative spheroids from test numbers 3-1 and 3-2 respectively. In FIGS. 13(a) and (b), the horizontal axis of the graph indicates the time (seconds). Further, the vertical axis indicates the measured fluorescence intensity as a ratio of the fluorescence intensity divided by the fluorescence intensity observed immediately following the start of measurements.

The results revealed that when the cell number ratio (neurons : astrocytes) was 1: 1, the expected type of change in the waveform of the oscillations was confirmed, whereas when the cell number ratio (neurons : astrocytes) was 4: 1, the expected type of change in the waveform of the oscillations could not be confirmed. Based on these results, it was clear that the cell number ratio (neurons : astrocytes) affected not only the spontaneous oscillations, but also the responsiveness to drugs.

### [Test Example 4]

### (Immunostaining of Neurons and Astrocytes in Spheroids)

Human iPSC-derived neurons (mixed neurons, manufactured by Elixirgen Scientific, Inc.) and human iPSC-derived astrocytes (Human iPS-derived Astrocytes (mature), manufactured by Xcell Science Inc.) were mixed so as to achieve a cell number of 8.0 × 10³ for each cell type per well.

Subsequently, each cell mixture was inoculated onto a spheroid production plate and cultured to produce a spheroid. A medium prepared by adding SM1 Supplement (manufactured by STEMCELL Technologies Inc.) and a 10% astrocyte culture supernatant (manufactured by ScienCell Research Laboratories, Inc.) to BrianPhys (manufactured by STEMCELL Technologies Inc.) was used as the medium.

Subsequently, spheroid immunostaining was conducted in the fifth week and the seventh week from the start of culturing. The spheroids were washed with phosphatebuffered saline (PBS), fixed with 4% paraformaldehyde, and then blocked with fetal bovine serum (BSA).

Subsequently, incubation with a primary antibody was conducted. For the primary antibodies, an anti-MPA2 antibody (610460, manufactured by BD Transduction Laboratories Inc.) which recognizes the neuron marker MAP2, and an anti-GFAP antibody (Z0334, manufactured by Dako Inc.) which recognizes the astrocyte marker GFAP were used.

Next, washing was performed, and incubation was then conducted with a secondary antibody. In accordance with the antibody used, an anti-mouse secondary antibody (a goat anti-mouse IgG (H+L) cross-adsorbed secondary antibody, Alexa Fluor 488, A11001, manufactured by Thermo Fisher Scientific Inc.) and an anti-rabbit secondary antibody (a goat anti-rabbit IgG (H+L) highly cross-adsorbed secondary antibody, Alexa Fluor 594, A11037, manufactured by Thermo Fisher Scientific Inc.) were used as the secondary antibodies.

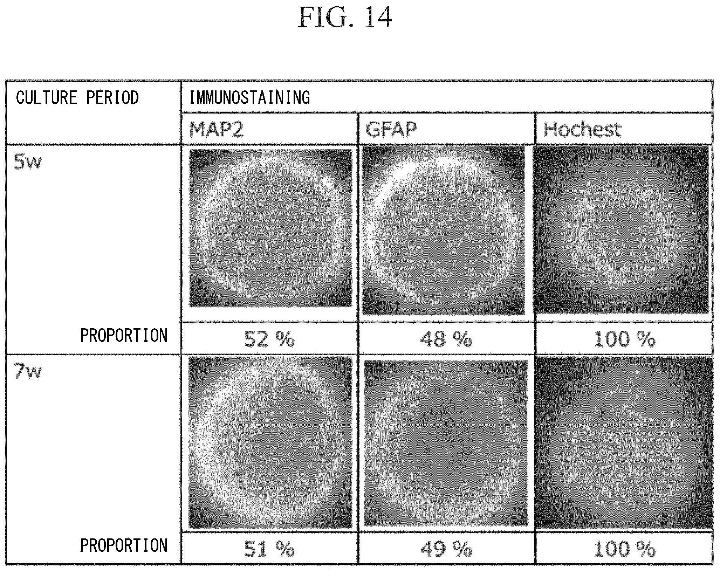

Subsequently, washing was performed, the nucleus was stained with Hoechst 33342 (manufactured by Thermo Fisher Scientific Inc.), and the spheroid was inspected using a fluorescence microscope. FIG. 14 is a series of fluorescence microscope photographs illustrating the results of the immunostaining. Each of the obtained images was analyzed using software (name: ImageJ, https://imagej.nih.gov/ij/), the number of MAP2 positive cells and the number of GFAP positive cells were calculated, and the proportions of neurons and astrocytes were determined. The proportion (%) of MAP2 positive cells, the proportion (%) of GFAP positive cells, and the proportion (%) of Hoechst 33342 positive cells are also shown in FIG. 14.

The results revealed that in both the spheroids in the fifth week from the start of culturing and the spheroids in the seventh week from the start of culturing, the cell number ratio (neurons : astrocytes) was approximately 1:1.

The present invention includes the following aspects.
[1] A cell-containing vessel comprising a plurality of neural cell-containing spheroids for which a variation calculated using a calculation method described below is less than 20%, wherein the neural cell-containing spheroids contain a plurality of types of cells including neural cells,
   (Calculation Method)
   Calcium transient assays are conducted for each of a plurality of neural cell-containing spheroids, a number of spontaneous oscillations in a 10-minute period is measured, an average and standard deviation for the number of spontaneous oscillations are calculated, and a variation is calculated using formula (1) below: Variation (%) = standard deviation for number of spontaneous oscillations / average number of spontaneous oscillations × 100
[2] The cell-containing vessel according to [1], wherein the neural cell-containing spheroids contain neurons and astrocytes, and a variation calculated using the calculation method described below is less than 15%,
   (Calculation Method)
   For each of a plurality of neural cell-containing spheroids, a cell number of neurons and a cell number of astrocytes are measured, an average and standard deviation for a ratio of the cell number of neurons relative to the cell number of astrocytes (cell number of neurons / cell number of astrocytes) are calculated, and a variation is calculated using formula (2) below: Variation (%) = standard deviation for (cell number of neurons / cell number of astrocytes) / average for (cell number of neurons / cell number of astrocytes) × 100
[3] The cell-containing vessel according to [2], wherein a variation calculated using a calculation method described below is less than 5%,
   (Calculation Method)
   For each of a plurality of neural cell-containing spheroids, a cell number of neurons and a cell number of astrocytes are measured, an average and standard deviation for a ratio of the cell number of neurons relative to the cell number of astrocytes (cell number of neurons / cell number of astrocytes) are calculated, and a variation is calculated using formula (2) below: Variation (%) = standard deviation for (cell number of neurons / cell number of astrocytes) / average for (cell number of neurons / cell number of astrocytes) × 100
[4] The cell-containing vessel according to [2] or [3], wherein in the neural cell-containing spheroids, the ratio of the cell number of neurons relative to the cell number of astrocytes (cell number of neurons / cell number of astrocytes) is approximately 1.
[5] The cell-containing vessel according to any one of [1] to [4], wherein the vessel is a multi-well plate, and contains one neural cell-containing spheroid in each well.
[6] A method for producing a neural cell-containing spheroid, the method including a step of mixing a plurality of types of neural cells in a predetermined ratio to form a spheroid, in which cell lineage of the neural cells has been determined.

The present invention may also include the following aspects.
[P1] A method for producing a neural cell-containing spheroid, the method including a step of mixing, in a predetermined ratio, a plurality of types of neural cells for which the cell lineage has been determined to form a spheroid.
[P2] A neural cell-containing spheroid containing a plurality of types of differentiated neural cells in a predetermined mixing ratio, wherein a variation calculated in accordance with a calculation method described below is less than 20%,
   (Calculation Method)
   Calcium transient assays are conducted for each of a plurality of neural cell-containing spheroids from across a plurality of lots or from within a single lot, a number of spontaneous oscillations in a 10-minute period is measured, an average and standard deviation for the number of spontaneous oscillations are calculated, and a variation is calculated using formula (1) below: Variation (%) = standard deviation for number of spontaneous oscillations / average number of spontaneous oscillations × 100
[P3] The neural cell-containing spheroid according to [P2], wherein the variation calculated in accordance with a calculation method described below is less than 15%,
   (Calculation Method)
   For each of a plurality of neural cell-containing spheroids from across a plurality of lots or from within a single lot, a cell number of neurons and a cell number of astrocytes are measured, an average and standard deviation for a ratio of the cell number of neurons relative to the cell number of astrocytes (cell number of neurons / cell number of astrocytes) are calculated, and a variation is calculated using formula (2) below: Variation (%) = standard deviation for (cell number of neurons / cell number of astrocytes) / average for (cell number of neurons / cell number of astrocytes) × 100
[P4] The neural cell-containing spheroid according to [P2], wherein the variation calculated in accordance with the calculation method described below is less than 5%,
   (Calculation Method)
   For each of a plurality of neural cell-containing spheroids from across a plurality of lots or from within a single lot, a cell number of neurons and a cell number of astrocytes are measured, an average and standard deviation for a ratio of the cell number of neurons relative to the cell number of astrocytes (cell number of neurons / cell number of astrocytes) are calculated, and the variation is calculated using formula (2) below: Variation (%) = standard deviation for (cell number of neurons / cell number of astrocytes) / average for (cell number of neurons / cell number of astrocytes) × 100
[P5] The neural cell-containing spheroid according to any one of [P2] to [P4], wherein the ratio of the cell number of neurons relative to the cell number of astrocytes (cell number of neurons / cell number of astrocytes) is 1.
[P6] A vessel containing the neural cell-containing spheroid according to any one of [P2] to [P5].
[P7] The vessel according to [P6], wherein the vessel is a multi-well plate, and contains one neural cell-containing spheroid in each well.

While preferred embodiments of the invention have been described and illustrated above, it should be understood that these are exemplary of the invention and are not to be considered as limiting. Additions, omissions, substitutions, and other modifications can be made without departing from the scope of the invention. Accordingly, the invention is not to be considered as being limited by the foregoing description and is only limited by the scope of the appended claims.

### PRIOR ART LITERATURE

### Patent Document

Patent Document 1: International Patent Publication WO2019/014603

## Claims

1. A method for producing a neural cell-containing spheroid, the method comprising the steps of:
mixing neurons for which the cell lineage has been determined and astrocytes for which the cell lineage has been determined in a predetermined ratio of cell number of neurons : cell number of astrocytes, wherein the predetermined ratio is within a range from 0.8 : 1 to 1.2 : 1; and
culturing a mixture obtained in the mixing step, thereby forming a spheroid.

2. The method for producing a neural cell-containing spheroid according to claim 1, wherein the neurons and astrocytes for which cell lineage has been determined are induced pluripotent stem (iPS) cells that are derived from a patient having a neural disorder or that have been subjected to gene editing so as to have genes that either cause, or are risk factors for, a neural disorder.

3. The method for producing a neural cell-containing spheroid according to claim 1, wherein the neurons for which cell lineage has been determined are pluripotent stem cells that have undergone a differentiation induction treatment to form neural cells.

4. The method for producing a neural cell-containing spheroid according to any one of claims 1 to 3,
wherein the predetermined ratio is 1 to 1.

## Patentansprüche

1. Verfahren zum Herstellen eines Nervenzellen enthaltenden Sphäroids, wobei das Verfahren die folgenden Schritte umfasst:
Mischen von Neuronen, für die die Zellabstammung bestimmt wurde, und Astrozyten, für die die Zellabstammung bestimmt wurde, in einem vorbestimmten Verhältnis von Zellanzahl von Neuronen : Zellanzahl von Astrozyten, wobei das vorbestimmte Verhältnis innerhalb eines Bereichs von 0,8:1 bis 1,2:1 liegt; und
Kultivieren einer in dem Schritt des Mischens erhaltenen Mischung, wodurch ein Sphäroid gebildet wird.

2. Verfahren zum Herstellen eines Nervenzellen enthaltenden Sphäroids nach Anspruch 1, wobei die Neuronen und die Astrozyten, für die eine Zellabstammung bestimmt wurde, induzierte pluripotente Stamm(iPS)-Zellen sind, die von einem Patienten mit einer Nervenstörung stammen oder die einer Genomeditierung unterzogen wurden, sodass sie Gene aufweisen, die entweder eine Nervenstörung verursachen oder Risikofaktoren dafür sind.

3. Verfahren zum Herstellen eines Nervenzellen enthaltenden Sphäroids nach Anspruch 1, wobei die Neuronen, für die eine Zellabstammung bestimmt wurde, pluripotente Stammzellen sind, die eine Differenzierungsinduktionsbehandlung durchlaufen haben, um Nervenzellen zu bilden.

4. Verfahren zum Herstellen eines Nervenzellen enthaltenden Sphäroids nach einem der Ansprüche 1 bis 3,
wobei das vorbestimmte Verhältnis 1 zu 1 beträgt.

## Revendications

1. Procédé de production d'un sphéroïde contenant des cellules neurales, le procédé comprenant les étapes consistant à :
mélanger des neurones pour lesquels la lignée cellulaire a été déterminée et des astrocytes pour lesquels la lignée cellulaire a été déterminée dans un rapport prédéterminé de nombre de cellules de neurones : nombre de cellules d'astrocytes, dans lequel le rapport prédéterminé est compris dans une plage allant de 0,8:1 à 1,2:1 ; et
mettre en culture un mélange obtenu lors de l'étape de mélange, formant ainsi un sphéroïde.

2. Procédé de production d'un sphéroïde contenant des cellules neurales selon la revendication 1, dans lequel les neurones et les astrocytes pour lesquelles une lignée cellulaire a été déterminée sont des cellules souches pluripotentes induites (iPS) qui proviennent d'un patient souffrant d'un trouble neuronal ou qui ont été soumises à une édition génétique de manière qu'elles présentent des gènes qui soit causent un trouble neuronal soit sont des facteurs de risque pour un tel trouble.

3. Procédé de production d'un sphéroïde contenant des cellules neurales selon la revendication 1, dans lequel les neurones pour lesquelles une lignée cellulaire a été déterminée sont des cellules souches pluripotentes qui ont subi un traitement d'induction de différenciation pour former des cellules neurales.

4. Procédé de production d'un sphéroïde contenant des cellules neurales selon l'une quelconque des revendications 1 à 3,
dans lequel le rapport prédéterminé est de 1 à 1.
